(19) Europäisches Patentamt
European Patent Office
Office européen des brevets

(11) Veröffentlichungsnummer: **0 150 263**
**B1**

(12) # EUROPÄISCHE PATENTSCHRIFT

(45) Veröffentlichungstag der Patentschrift: **20.06.90**

(21) Anmeldenummer: **84111056.2**

(22) Anmeldetag: **02.11.82**

(60) Veröffentlichungsnummer der früheren Anmeldung nach Art. 76 EPÜ: **0 079 022**

(51) Int. Cl.[5]: **C 07 D 209/52**, C 07 C 229/28, C 07 C 229/36, A 61 K 31/40

(54) **Derivate der cis, endo-2-Azabicyclo[3.3.0]octan-3-carbonsäure, Verfahren zu ihrer Herstellung, diese enthaltende Mittel und deren Verwendung.**

(30) Priorität: **05.11.81 DE 3143946**
**17.07.82 DE 3226768**

(43) Veröffentlichungstag der Anmeldung:
**07.08.85 Patentblatt 85/32**

(45) Bekanntmachung des Hinweises auf die Patenterteilung:
**20.06.90 Patentblatt 90/25**

(84) Benannte Vertragsstaaten:
**AT BE CH DE FR GB IT LI LU NL SE**

(56) Entgegenhaltungen:
**EP-A- 90 362**
**EP-A-0 037 231**
**DE-B-1 955 375**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT**
**Postfach 80 03 20**
**D-6230 Frankfurt am Main 80 (DE)**

(72) Erfinder: **Teetz, Volker, Dr.**
**An der Tann 20**
**D-6238 Hofheim am Taunus (DE)**
Erfinder: **Geiger, Rolf, Prof. Dr.**
**Heinrich-Bleicher-Strasse 33**
**D-6000 Frankfurt am Main 50 (DE)**
Erfinder: **Urbach, Hansjörg, Dr.**
**Le Lavandoustrasse 41**
**D-6242 Kronberg/Taunus (DE)**
Erfinder: **Becker, Reinhard, Dr.**
**Adelheidstrasse 101**
**D-6200 Wiesbaden (DE)**
Erfinder: **Schölkens, Bernward, Dr.**
**Am Fliedergarten 1**
**D-6233 Kelkheim (Taunus) (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 150 263 B1

(56) Entgegenhaltungen:

**CHEMICAL ABSTRACTS, Band 89, Nr. 25, 18. Dezember 1978, Columbus, Ohio, USA; S.G. AGBALYAN et al. "Addition of aliphatic amino acids to beta-aroylacrylic acids", Seite 630, rechte Spalte, Zusammenfassung Nr. 215732p & Arm. Khim. Zh., Band 31, Nr. 4, 1978, Seiten 273**

**CHEMICAL ABSTRACTS, Band 88, Nr. 5, 30. Januar 1978, Columbus, Ohio, USA; Y. MORIKAWA et al. "Beta-Benzoylacrylic acid", Seite 470, linke Spalte, Zusammenfassung Nr. 37442p & JP-A-77-39020 (Y. MORIKAWA et al.)**

**CHEMICAL ABSTRACTS, Band 89, Nr. 15, 9. Oktober 1978, Columbus, Ohio, USA; C. LINZ A.-G. "3-Phenyl-6-pyridazone", Seite 595, linke Spalte, Zusammenfassung Nr. 129529w & JP-A-77-116485 (CHEMIE LINZ A.-G.)**

**Beschreibung**

Derivate der cis,endo-2-Azabicyclo[3.3.0]octan-3-carbonsäure, Verfahren zu ihrer Herstellung, diese enthaltende Mittel und deren Verwendung.

Aus Chem. Abstr. *89* [1978] 215 732 p ist ein Verfahren zur Herstellung von β-Aroyl-α-(N-succinyl-aminopropionsäuren durch Umsetzung von β-Aroylarylsäuren mit Asparaginsäure in Gegenwart von Kaliumhydroxid bekannt.

EP—A—0 090 362 betrifft u.a. Octahydrocyclopenta[c]pyrrol-1-carbonsäure-Derivate und deren Verwendung als Hemmer des Angiotensin-Convesting-Enzyme (ACE).

Es wurden neue cis,endo-2-Azabicyclo[3.3.0]octan-3-carbonsäure-Derivate gefunden, die das ACE stark hemmen und die eine langdauernde, intensive blutdrucksenkende Wirkung besitzen.

Die Erfindung betrifft daher cis,endo-2-Azabicyclo[3.3.0]octan-3-carbonsäure-Derivate der Formel I

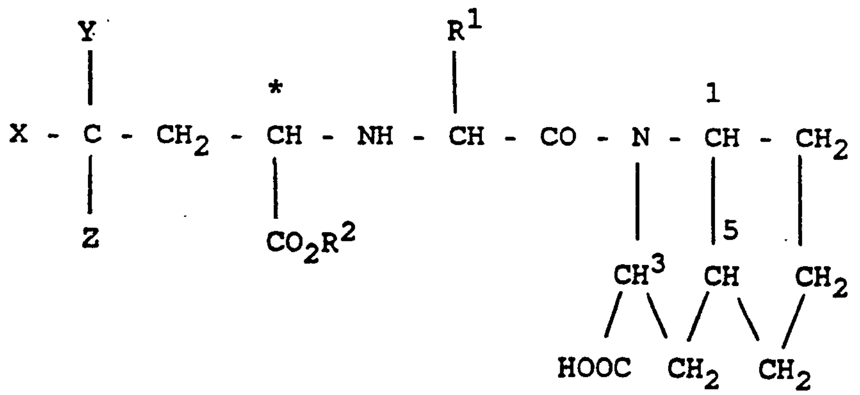

(I)

in der die Wasserstoffatome an den Brückenkopf-C-Atomen 1 und 5 zueinander cis-konfiguriert sind und die Carboxygruppe am C-Atome 3 endoständig zum bicyclischen Ringsystem orientiert (d.h. dem Cyclopentanring zugewandt) ist und in der

$R^1$ Wasserstoff, Allyl, Vinyl oder eine Seitenkette einer gegebenenfalls geschützten natürlich vorkommenden α-Aminosäure $R^1$—CH($NH_2$)—COOH;

$R^2$ Wasserstoff, ($C_1$—$C_6$)-Alkyl, ($C_2$—$C_6$)-Alkenyl, Aryl-($C_1$—$C_4$)-alkyl oder Nitrobenzyl,

Y Wasserstoff oder Hydroxy,

Z Wasserstoff oder

Y und Z zusammen Sauerstoff,

X ($C_1$—$C_6$)-Alkyl, ($C_2$—$C_6$)-Alkenyl, ($C_5$—$C_9$)-Cycloalkyl, ($C_6$—$C_{12}$)-Aryl, das durch ($C_1$—$C_4$)-Alkyl, ($C_1$—$C_4$)-Alkoxy, Hydroxy, Halogen, Nitro, Amino, ($C_1$—$C_4$)-Alkylamino, Di-($C_1$—$C_4$)-alkyl-amino oder Methylendioxy mono-, di- oder trisubstituiert sein kann, oder Indol-3-yl bedeuten,

sowie deren physiologisch unbedenklichen Salze, wobei Verbindungen der Formel I und deren Salze ausgenommen sind, in der die Chiralitätszentren an dem mit einem Stern (*) markierten beiden C-Atomen der Kette und am C-Atom 3 des Bicyclus in der S-Konfiguration vorliegen und in der $R^1$ Methyl, $R^2$ Wasserstoff, Methyl, Ethyl oder Benzyl, Y Wasserstoff, Z Wasserstoff und X Phenyl bedeuten.

Bevorzugt sind Verbindungen der Formel I, in der $R^1$ Methyl, die gegebenenfalls acyclierte Seitenkette von Lysin oder die O-alkylierte Seitenkette von Tyrosin, $R^2$ Wasserstoff, Methyl, Ethyl oder Benzyl, X Phenyl oder mit Fluor und/oder Chlor mono- oder disubstituiertes Phenyl, Y Wasserstoff oder Hydroxy, Z Wasserstoff oder Y und Z zusammen Sauerstoff bedeuten.

Falls $R^1$ für eine Seitenkette einer geschützten natürlichen vorkommenden α-Aminosäure steht, wie z.B. geschütztes Ser, Thr, Asp, Asn, Glu, Gln, Arg, Lys, Hyl, Cys, Orn, Cit, Tyr, Trp oder His, so sind als Schutzgruppen die in der Peptidchemie üblichen Gruppen bevorzugt (vgl. Houben-Weyl, Bd. XV/1 und XV/2). Im Falle, daß $R^1$ die geschützte Lysin-Seitenkette bedeutet, werden die bekannten Amino Schutzgruppen, inbesondere aber ($C_1$—$C_6$)-Alkanoyl bevorzugt. Als O-Schutzgruppen für Tyrosin kommen bevorzugt Methyl und Ethyl in Frage.

Als Salze kommen insbesondere in Frage die Hydrochloride, Maleinate, Tartrate bzw. die Alkyl-, Ca-, Mg- und Zn-Salze.

Die Chiralitätszentren an dem mit einem Stern (*) markierten C-Atomen der Kette und an C-Atom 3 des Bicyclus können sowohl die R- als auch die S-Konfiguration haben. Bevorzugt sind jedoch Verbindungen, in denen diese Zentren in der S-Konfiguration vorliegen. Falls —NH—*CH$R^1$—CO— für Cys steht, ist jedoch die R-Konfiguration dieses Zentrums bevorzugt.

Gegenstand der Erfindung ist ferner ein Verfahren zur Herstellung der Verbindungen der Formel I, das dadurch gekennzeichnet ist, daß man eine Verbindung der Formel II, in der X, Y, Z und $R^1$ wie oben definiert sind und $R^2$ die vorstehend genannten Bedeutungen mit Ausnahme der von Wasserstoff hat, mit einer Verbindung der Formeln IIIa oder IIIb, in denen W eine Carboxy veresternde Gruppe, wie ($C_1$—$C_6$)-Alkyl oder ($C_7$—$C_8$)-Aralkyl, vorzugsweise tert.-Butyl oder Benzyl bedeutet, nach bekannten Amidbildungsmethoden der Peptidchemie umsetzt und anschließend durch Hydrierung oder Säure- oder/und Basenbehandlung die Verbindungen des Typs I freisetzt und die erhaltenen Verbindungen gegebenenfalls in ihre physiologisch verträglichen Salze überführt.

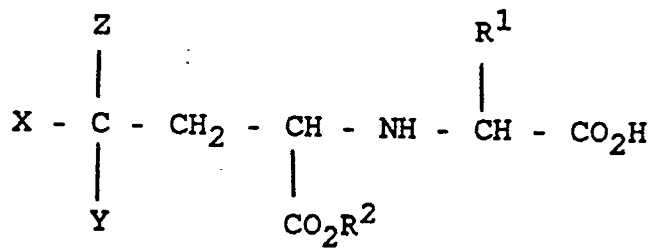

(II)

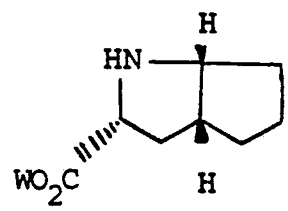

(IIIa)

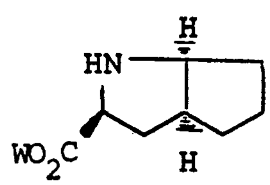

(IIIb)

Verbindungen der Formel II mit X = Phenyl, Y = H, Z = H und $R^2$ = $CH_3$ oder $C_2H_5$ sind bekannt (z.B. aus der EP—A—0037321) und auf verschiedenen Wegen zugänglich. Die Benzylester ($R^2$ = Benzyl) können analog hergestellt werden.

Es wurde ferner gefunden, daß die Mannich-Reaktion von Acetophenonen der Formel IVa, in der X für gegebenenfalls wie vorstehend substituiertes Aryl steht mit Glyoxylsäureestern und α-Aminosäureestern zu Verbindungen der Formel II führt, in denen Y und Z zusammen Sauerstoff bedeuten (Formel IV). Bei der Hydrogenolyse dieser Verbindungen mit Pd entstehen Verbindungen der Formel II, in denen Y und Z Wasserstoff sind.

Verbindungen der Formel II in der Y und Z zusammen Sauerstoff bedeuten, können ebenfalls durch Michael-Addition entsprechender Keto-Acrylsäureester mit α-Aminosäureestern in hohen Ausbeuten gewonnen werden. Esterspaltung führt zu denselben Produkten wie die Mannich-Reaktion.

Die Erfindung betrifft daher auch ein Verfahren zur Herstellung von Verbindungen der Formel II, in welcher X, $R^1$ und $R^2$ die oben definierten Bedeutungen haben, Y Wasserstoff oder Hydroxy und Z Wasserstoff bedeuten, das dadurch gekennzeichnet ist, daß man

a) falls X Aryl ist, Arylmethylketone X—CO—$CH_3$ mit Glyoxylsäureestern CHO—$CO_2R^2$ und α-Aminosäureestern $H_2N$—CH($R^1$)—$CO_2W'$, in welchen W' ein hydrogenolytisch, basisch oder sauer abspaltbarer Rest, bevorzugt Benzyl oder tert.-Butyl ist und $R^2$ für den Fall W' = Benzyl, nicht Benzyl oder Nitrobenzyl bedeutet, zu Verbindungen der Formel IV umsetzt.

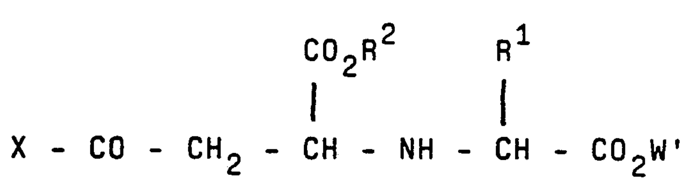

(IV)

in der X, W', $R^1$ und $R^2$ die vorstehend abgegebenen Bedeutungen haben, oder

b) Keto-Acrylsäureester X—CO—CH=CH—$CO_2R^2$ mit α-Aminosäureestern $H_2N$—CH($R^1$)—$CO_2W'$ zu Verbindungen der Formel IV umsetzt,

diese nach a) oder b) erhaltenen Verbindungen, falls W' ein sauer oder basisch abspaltbarer Rest ist, gegebenenfalls einer sauren oder basischen Esterspaltung unterwirft oder, falls W' = Benzyl oder Nitrobenzyl ($R^2$ ≠ Benzyl oder Nitrobenzyl) hydrogenolytisch in die Carbonsäure überführt und zu

Verbindungen der Formel II, in der der X, $R^1$ und $R^2$ die vorstehende Bedeutung hat, Y Wasserstoff oder Hydroxy und Z Wasserstoff bedeuten, beziehungsweise deren, Niederalkylestern hydriert, welche gegebenenfalls auch einer sauer katalysierten Enterspaltung unterworfen werden können.

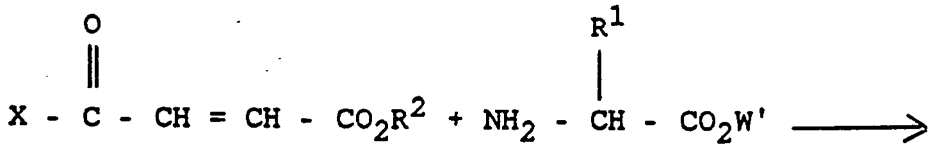

IV

Die Diastereomeren mit der bevorzugten S,S-Konfiguration entstehen bei Einsatz von S-Alaninestern in überwiegender Menge und können durch Kristallisation oder chromatographische Trennung der Ester von II an Kieselgel gewonnen werden.

Die neuen Verbindungen der Formel I besitzen eine langdauernde, intensive blutdrucksenkende Wirkung. Sie sind starke Hemmer des Angiotensin-Converting-Enzyms (ACE-Hemmer) und können zur Bekämpfung des Bluthochdrucks verschiedener Genese eingesetzt werden. Auch ihre Kombination mit anderen blutdrucksenkenden, gefäßerweiternden oder diuretisch wirksammen Verbindungen sind möglich. Typische Vertreter dieser Wirkklassen sind z.B. in Erhardt-Ruschig, Arzneimittel, 2. Auflage Weinheim, 1972, beschrieben. Die Anwendung kann intravenös, subcutan oder peroral erfolgen.

Die Dosierung bei oraler Gabe liegt bei 1—100 mg je Einzeldosis. Die kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden.

Die erfindungsgemäßen Verbindungen können oral oder parenteral in entsprechender pharmazeutischer Zubereitung verabreicht werden. Für eine orale Anwendungsform werden die aktiven Verbindungen mit den dafür übglichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesiumcarbonat, Karliumphosphat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträglichen Salze, gewünschtenfalls mit den dafür üblichen Substanzen wie Lösungsvermittler, Emulgatoren oder weiteren Hilfsstoffe in Lösung, Suspension oder Emulsion gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propandiol oder Glycerin, daneben auch Zuckerlösungen wie Glucose- oder Mannitlösungen, oder auch eine Mischung aus den verschiedenen genannten Lösungsmitteln.

Die außerordentlich starke Wirksamkeit oder Verbindungen gemäß Formel I — auch bei oraler Gabe — wird durch nachfolgende pharmakologische Daten belegt.

Intraduodenale Gabe an der narkotisierten Ratte, 50% Hemmung der durch 310 ng Angiotensin I ausgelösten Pressorreaktion 30 Min. nach Applikation in der Dosis ... $ED_{50}$.

| X | Y | Z | $R^1$ | $R^2$ | $ED_{50}$ (μg/kg) |
|---|---|---|---|---|---|
| $C_6H_5$ | - | O | $CH_3$ | $CH_3$ | 350 |
| $C_6H_5$ | - | O | $CH_3$ | $C_2H_5$ | 280 |
| $C_6H_5$ | - | O | $CH_3$ | H | 720 |
| $C_6H_5$ | - | O | $CH_3$ | $C_7H_7$ | 250 |
| $C_6H_5$ | H | OH | $CH_3$ | $C_2H_5$ | 380 |
| p-Cl-$C_6H_4$ | H | H | $CH_3$ | $C_2H_5$ | 55 |
| p-Cl-$C_6H_4$ | - | O | $CH_3$ | H | 780 |

Die nachfolgenden Beispiele sollen die erfindungsgemäßen Verfahrensweisen erläutern, ohne die Erfindung auf die hier stellvertretend genannten Substanzen zu beschränken.

Referenzbeispiel
2-[N-(1-S-Carboethoxy-3-phenyl-propyl)-S-alanyl]-2-cis,endo-azbicyclo[3.3.0]octan-3-S-carbonsäure

(1) 2-Acetylamino-3-(2-oxo-cyclopentyl)-propionsäuremethylester:

269 g 3-Chlor-2-acetyl-amino-propionsäuremethylester und 257 g Cyclopentenopyrrolidin werden in 1,5 l DMF 24 Stunden bei Raumtemperatur gehalten. Man engt im Vakuum ein, nimmt den Rückstand in wenig Wasser auf, stellt mit konzentrierter Salzsäure auf pH 2 und extrahiert 2 mal jit je 4 l Essigester. Beim Einengen der organischen Phase hinterbleibt ein hellgelbes Öl.

Ausbeute: 290 g.

NMR: 2,02 (s, 3H); 3,74 (2, 3H); 4,4—4,8 (m, 1H) ($CDCl_3$)

| Analyse: | C | H | N |
|---|---|---|---|
| ber. | 58,1 | 7,54 | 6,16 |
| gef. | 58,5 | 7,2 | 6,5 |

(2) cis,endo-2-Azabicyclo[3.3.0]octan-3-carbonsäurehydrochlorid

270 g des unter (1) hergestellten Acetylamino-Derivatives werden in 1,5 l 2 n Salzsäure 45 Minuten am Rückfluß gekocht. Man engt im Vakuum ein, nimmt den Rückstand in Eisessig auf, versetzt mit 5 g Pt/C (10% Pt) und hydriert bei 5 bar. Nach Filtration wird eingeengt und der Rückstand aus Chloroform/Diisopropylether kristallisiert.

Schmelzpunkt: 205—209°C.

Ausbeute: 150g.

(3) cis,endo-2-Azabicyclo[3.3.0]octan-3-carbonsäurebenzylester-hydrochlorid

40 g der unter (2) hergestellten Carbonsäure werden in eine eiskalte Mischung aus 390 g Benzylalkohol und 65 g Thionylchlorid gegeben und 24 Stunden bei Raumtemperatur belassen. Nach Einengen im Vakuum kristallisieren 47 g des Benzylesters aus Chloroform/Isopropanol.

Schmelzpunkt: 175°C (Hydrochlorid)

(4) 2-[N-(1-S-Carbethoxy-3-phenyl-propyl)-S-alanyl]-cis,endo-2-azabicyclo[3.3.0]octan-3-S-carbonsäurebenzylester

14 g des nach (3) hergestellten Benzylesters werden mit 6,7 g HOBt, 13,8 g N-(1-S-Carbethoxy-3-phenyl-propyl)-S-alanin, und 10,2 g Dicyclohexylcarbodiimid in 200 ml Dimethylformamid zur Reaktion gebracht. Nach 3 Stunden Rühren bei Raumtemperatur saugt man von ausgefallenem Dicyclohexyharnstoff ab, engt ein, nimmt in 1 l Essigester auf und schüttelt mit 3 × 500 ml 5-prozentiger $NaHCO_3$-Lösung aus. Die organische Phase wird eingeengt und mit Essigester/Petrolether im Verhältnis 2:1 über eine Säule aus 1 kg Kieselgel chromatographiert. Das zuerst eluierte Isomere stellt die S,S,S-Verbindung dar, ein späteres Eluat liefert nach dem Einengen die S,S,R-Verbindung.

Es werden Jeweils 8,0 g Produkt als Öl erhalten.

NMR: der S,S,S-Verbindung: Charakteristische Signale: 1,20 (d, 3H), 1,27 (t, 2H), 4,17 (q, 3H), 5,13 (s 2H), 7,18 (s, 5H), 7,32 (s, 5H) ($CDCl_3$)

| Analyse: | | C | H | N |
|---|---|---|---|---|
| $C_{30}H_{38}N_2O_5$ | ber. | 71,1 | 7,56 | 5,53 |
| | gef. | 70,8 | 7,8 | 5,7 |

(5) 2-[N-(1-S-Carbethoxy-3-phenyl-propyl)-S-alanyl]-cis,endo-2-azabicyclo[3.3.0]octan-3-S-carbonsäure

8,0 g des L,L,L-Benzylesters aus (4) werden in 100 ml Äthanol gelöst und unter Zusatz von 0,5 g 10% Pd/C bei Normaldruck hydrogenolisch entbenzyliert. Diese Reaktion kann auch unter Druck bei gleichzeitiger Verkürzung der Reaktionszeit vorgenommen werden. Nach Aufnahme der berechneten Menge Wasserstoff wird vom Katalysator abfiltriert und im Vakuum eingeengt. Das Zwitterion kristallisiert in fast quantitativer Ausbeute aus Ether:

Schmelzpunkt: 110—112°C (Zers.)

Durch Zusatz einer äquivalenten Menge Salzsäure kann ein Hydrochlorid (ab 120°C Zersetzung) oder durch Zugabe von wäßrigen Zinksalzen zu einer konzentrierten methanolischen Lösung der Titelverbindung ein thermisch besonders stabiles Zink-Komplexsalz (Zersetzung über 160°C) erhalten werden.

| Analyse: | | C | H | N |
|---|---|---|---|---|
| $C_{23}H_{32}N_2O_5$ | ber. | 66,3 | 7,7 | 6,73 |
| | gef. | 66,1 | 7,8 | 6,6 |

Die erhaltenen NMR— und Massenspektren sind im Einklang mit der angegebenen Struktur.
$[\alpha]_D = +15,6°$ (c = 1, Methanol).

Beispiel 1

(1) cis,endo-2-Azabicyclo[3.3.0]octan-3-carbonsäure-tert.-butylester

25 g Azabicyclo[3.3.0]octancarbonsäure-hydrochlorid aus dem Referenzbeispiel werden in 250 ml Dioxan mit 250 ml Isobutylen und 25 ml konzentrierter Schwefelsäure zur Reaktion gebracht. Nach 14 Stunden bei Raumtemperatur wird mit Natronlauge alkalisch gestellt, im Vakuum eingeengt, mit 100 ml Wasser versetzt und der Ester ausgeethert. Nach Abdampfen des Ethers erhält man 15 g farbloses Öl.

| Analyse: | | C | H | N |
|---|---|---|---|---|
| $C_{12}H_{21}NO_2$ | ber. | 68,2 | 10,2 | 6,63 |
| | gef. | 67,9 | 10,1 | 6,3 |

(2) N-(1-S-Carbobenzyloxy-3-oxo-3-phenyl-propyl)-S-alanin-tert.-butylester

12,0 g Acetophenon, 17 g Glyoxylsäurebenzylester und 31,7 g Alanin-tert.-butylester-toluolsulfonat werden 200 ml Eisessig auf 45—50°C 24 bis 48 Stunden erhitzt. Die Reaktion wird dünnschicht-chromatographisch verfolgt und am optimalen Umsetzungspunkt abgebrochen. Man engt im Vakuum gut ein, stellt mit wäßriger Bicarbonatlösung basisch und extrahiert mit Essigester. Man engt die organische Phase möglichst weitgehend ein und kristallisiert das S,S-Isomere aus Cyclohexan/Petrolether. Die R,S-Verbindung bleibt weitgehend in Lösung. Zum Erhalt von Impfkristallen empfiehlt sich eine Chromatographie des Rohgemisches an Kieselgel im System Cyclohexan/Essigester 2:1 dem man 0,1% Triäthylamin zusetzt. Die S,S-Verbindung wird als zweite der beiden Diastereomeren eluiert und fällt in größerer Menge an. Man erhält 9 g.

| Analyse: | | C | H | N |
|---|---|---|---|---|
| $C_{24}H_{29}NO_5$ | ber. | 70,1 | 7,1 | 3,4 |
| | gef. | 70,0 | 6,9 | 3,5 |

(3) N-(1-S-Carbobenzyloxy-3-oxo-3-phenyl-propyl)-S-alanintrifluoracetat

8 g des Mannich-Kondensationsproduktes aus I (2) werden in 25 ml wasserfreiem Trifluoressigsäure gelöst und eine Stunde bei Raumtemperatur belassen. Man engt im Vakuum ein, versetzt mit Diisopropylether und fällt mit Petrolether. Man erhält 7,2 g amorphe Substanz.

| Analyse: | | C | H | N |
|---|---|---|---|---|
| $C_{22}H_{22}NO_7F_3$ | ber. | 56,3 | 4,7 | 3,0 |
| | gef. | 56,0 | 4,8 | 3,1 |

MW: 469

(4) 2-[N-(1-S-Carbobenzyloxy-3-oxo-3-phenyl-propyl)-S-alanyl]-2-cis,endo-azabicyclo[3.3.0]octan-3-carbonsäure-tert.-butylester

35,5 g des N-substituierten Alanins aus I (3) reagiert mit 21,1 g Azabicyclooctancarbonsäure-tert.-butylester aus Beispiel I (1) analog Referenzbeispiel (4). Man erhält nach Chromatographie über Kieselgel 20,3 g der Titelverbindung.

| Analyse: | | C | H | N |
|---|---|---|---|---|
| $C_{32}H_{40}N_2O_6$ | ber. | 70,04 | 7,35 | 5,10 |
| | gef. | 69,6 | 7,4 | 5,3 |

(5) 2-[N-(1-S-Carbobenzyloxy-3-oxo-3-phenyl-propyl)-S-alanyl]-2-cis,endo-azabicyclo[3.3.0]octan-3-carbonsäure

20 g des tert.-Butylesters aus I (4) werden in 100 ml TFA gelöst und eine Stunde bei Raumtemperatur belassen.

Man engt im Vakuum ein, nimmt das hinterbleibende Harz in Essigester auf und neutralisiert mit wäßrigem Bicarbonat. Aus der Essigesterphase werden 14 g der Titelverbindung gewonnen.

| Analyse: | | C | H | N |
|---|---|---|---|---|
| $C_{28}H_{32}N_2O_6$ | ber. | 68,27 | 6,55 | 5,69 |
| | gef. | 68,1 | 6,4 | 5,7 |

(6) 2-[N-(1-S-Carboxy-3-R,S-hydroxy-3-phenyl-propyl)-S-alanyl]-cis,endo-azabicyclo[3.3.0]octan-3-carbonsäure

1 g 2-[N-(1-S-Carbobenzyloxy-3-oxo-3-phenyl-propyl-S-alanyl]-cis,endo-2-azabicyclo[3.3.0]octan-3-carbonsäure werden in 50 ml Ethanol gelöst, mit 150 mg $Pd/BaSO_4$ versetzt und bei Normaldruck hydriert. Nach Aufnahme der berechneten Wasserstoffmenge wird filtriert, eingeengt und über Kieselgel im Solvens $CHCl_3/CH_3OH/CH_3COOH$ 50:20:2 chromatographiert.

Ausbeute: 0,6 g

(7) 2-[N-(1-S-Carbobenzyloxy-3-R,S-hydroxy-3-phenyl-propyl)-S-alanyl]-cis,endo-2-azabicyclo[3.3.0]octan-3-carbonsäure

1 g 2-[N-(1-S-Carbobenzyloxy-3-oxo-3-phenyl-propyl)-S-alanyl]-2-cis,endo-2-azabicyclo[3.3.0]octan-3-carbonsäure werden in 50 ml eines Gemisches aus Acetonitril und Wasser gelöst und mit 150 mg $NaBH_4$ reduziert. Nach 12 Stunden wird zur Trockene eingeengt, mit verdünnter Salzsäure neutral gestellt und die Titelverbindung mit Essigester extrahiert. Zur Entfernung von Borsäure und anderen Verunreinigungen wird über Kieselgel im Solvens $CHCl_3$—$CH_3OH$—$CH_3COOH$ 50:10:5 chromatographiert.

| Analyse: | | C | H | N |
|---|---|---|---|---|
| $C_{28}H_{34}N_2O_6$ | ber. | 67,99 | 6,93 | 5,66 |
| | gef. | 67,7 | 6,6 | 5,3 |

Algemeine Methode zur Darstellung von Verbindungen der Formel I mit $R^2$ = H durch Esterverseifung.

10 g des entsprechenden Ethyl- oder Benzylesters der Formel I werden in 200 ml Dimethoxyethan gelöst. Man fügt einen Tropfen einer verdünnten Indikatorlösung, z.B. Bromthymolblau, zu und fügt unter starkem Rühren im Verlauf von 5 Minuten eine äquivalente Menge 4n KOH (wäßrig) hinzu, so daß der Indikator bei Beendigung der Reaktion einen pH-Wert von 9—10 anzeigt. Sodann stellt man mit Salzsäure auf pH 4, engt im Vakuum zur Trockene ein, nimmt in 250 ml Esigester auf und filtriert. Beim Einengen des Essigesters fallen die Dicarbonsäuren als feste, kristalline oder amorphe Verbindungen an.

Die Ausbeuten liegen zwischen 80 und 95%.

Beispiel II

N-(1-S-Carbethoxy-3-oxo-3-phenyl-propyl)-S-alanin-benzylester

65,7 g 3-Phenyl-3-oxo-1-propen-1-carbonsäureethylester (Benzoylacrylsäureethylester) werden in 225 ml Ethanol gelöst und dazu 1 ml Triethylamin gegeben. Zu dieser Lösung wird bei Raumtemperatur eine Lösung von 70 g S-Alaninbenzylester in 90 ml Ethanol rasch zugetropft. Es wird 2 Stunden bei Raumtemperatur gerührt und dann die Lösung angekühlt. Es kristallisiert das S,S-Isomere aus.

Ausbeute: 94,3 g Fp: 73—74°C

$[\alpha]_D^{20} = +17,8°$ (c = 1, $CH_3OH$)

Beispiel III

N-(1-S-Carbethoxy-3-oxo-3-phenyl-propyl)-S-alanin

0,5 g der Verbindungen aus Beispiel II werden in 40 ml Ethanol gelöst und 0,1 g Pd/C 10%ig zugegeben und bei Raumtemperatur und Normaldruck hydriert.

Ausbeute: 300 mg Fp: 210—220°C

$^1H$—NMR (DMSO-$d_6$): 1,0—1,4 (t, 6H); 3,2—5,0 (m, 8H); 7,2—8,2 (m, 5H)

Beispiel IV

2-[N-(1-S-Carbethoxy-3-oxo-3-phenyl-propyl)-S-alanyl]-cis,endo-2-azabicyclo[3.3.0]octan-3-S-carbonsäurebenzylester

Die Verbindung wird aus cis,endo-2-Azabicyclo[3.3.0]-octan-3-S-carbonsäurebenzylester-hydrochlorid und N-(1-S-Carbethoxy-3-oxo-3-phenyl-propyl)-S-alanin aus Beispiel III analog dem Verfahren, das im Referenzbeispiel (4) beschrieben ist, hergestellt.

Beispiel V

2-[N-(1-S-Carbethoxy-3-oxo-3-phenyl-propyl)-S-alanyl]-cis,endo-2-azabicyclo[3.3.0]octan-3-S-carbonsäure

1 g des Benzylesters aus Beispiel IV werden in 30 ml Ethanol gelöst, mit 100 mg Pd/C (10%ig) bei Raumtemperatur und Normaldruck hydriert. Nach der Aufnahme eines Moläquivalentes Wasserstoff wird die Hydrierung abgebrochen. Es wird vom Katalysator abgesaugt, die Lösung eingeengt.

Ausbeute: 600 mg Öl.

$^1H$—NMR (DMSO-$d_6$): 1,0—3,0 (m, 15H) 3,3—5,0 (m, 10H); 7,2—8,1 (m, 5H)

Beispiel VI

2-[Nα-(1-S-Carbethoxy-3-phenyl-propyl)-S-lysyl]-cis,endo-2-azabicyclo[3.3.0]octan-3-S-carbonsäure-dihydrochlorid

(1) Nα-(1-S-Carbethoxy-3-oxo-3-phenyl-propyl)-$N_\varepsilon$-benzyloxycarbonyl-S-lysinbenzylester

10 g 3-Phenyl-3-oxo-1-propen-1-carbonsäureethylester werden in 100 ml Ethanol gelöst. Dazu werden 19,1 g $N_\varepsilon$-Benzyloxycarbonyl-S-lysinbenzylester und 0,2 g Triethylamin gegeben. Die Lösung wird 3 Stunden bei Raumtemperatur gerührt, danach im Vakuum eingeengt. Der ölige Rückstand (31 g) wird in Isopropanol/Diisopropylether gelöst und abgekühlt. Es kristallisieren 13 g $N_\alpha$-(1-S-Carbethoxy-3-oxo-3-phenyl-propyl)-$N_\varepsilon$-benzyloxycarbonyl-S-lysinbenzylester.

$\alpha_D^{20} = 3,5°$ (c = 1, $CH_3OH$)

$^1H$—NMR ($CDCl_3$): 1,0—1,4 (tr, 3H) 1,0—2,0 (m, 9H); 2,0—2,6 (breites s., 1H); 2,9—3,9 (m, 6H); 3,9—4,4 (quadr., 2H); 4,6—4,9 (breites s., 1H); 5,0—5,2 (doppeltes s, 4H); 7,1—8,1 (m, 15 H)

(2) Nα-(1-S-Carbethoxy-3-phenyl-propyl)-$N_\varepsilon$-benzyloxycarbonyl-S-lysin

4,0 g des in Beispiel VI (1) hergestellten Lysinbenzylesterderivates werden in 50 ml Eisessig gelöst, dazu 0,6 g Pd/C (10%ig) und 0,6 g konz.

Schwefelsäure gegeben. Es wird 6 Stunden bei Raumtemperatur und unter Normaldruck hydriert. Danach wird vom Katalysator abgesaugt, die ethanolische Lösung mit 1,4 g festem Natriumhydrogencarbonat gerührt. Die Lösung wird einrotiert und der Rückstand in Wasser gelöst. Die wäßrige Phase wird mit Essigester und Methylenchlorid extrahiert. Die organischen Phasen werden verworfen und die wäßrige Phase im Vakuum zur Trockne eingedampft. Der Rückstand wird mit Methanol ausgerührt. Nach dem Abdampfen des Methanol bleibt ein Öliger Rückstand, der bei Behandlung mit Diisopropylether fest wird. Ausbeute an $N_\alpha$-(1-S-Carbethoxy-3-phenyl-propyl)-S-lysin: 2,0 g.

$^1$H—NMR ($D_2O$): 1,0—1,4 (tr, 3H); 1,0—2,5 (m, 9H); 2,5—4,4 (m, 9H); 3,9—4,4 (q, 2H); 4,5—5,0 (m, 1H); 7,1—7,6 (m, 5H)

m/e: 336

3,4 g $N_\alpha$-(1-S-Carbethoxy-3-phenyl-propyl)-S-lysin werden in 30 ml Methylenchlorid gelöst und auf 0°C abgekühlt. Dazu werden unter Eiskühlung 2,1 g Triethylamin gegeben und anschließend 1,9 g Chlorameisenbenzylester zugetropft. Es wird 1 Stunde bei 0°C gerührt und dann auf Raumtemperatur gebracht. Die Methylenchloridlösung wird nacheinan der mit Wasser, Natriumcarbonatlösung und Wasser ausgeschüttelt. Nach dem Trocknen wird eingeengt und der ölige Rückstand über Kieselgel mit Methylenchlorid/Methanol chromatographiert. Es werden 2,0 g $N_\alpha$-(1-S-Carbethoxy-3-phenyl-propyl)-$N_\varepsilon$-benzyloxycarbonyl-S-lysin erhalten.

$^1$H—NMR ($D_2O$): 1,0—1,4 (tr, 3H); 1,0—2,5 (m, 9H); 2,5—4,4 (m, 9H); 3,9—4,4 (q, 2H); 4,5—5,0 (m, 1H); 5,1 (s, 2H); 7,1—7,5 (m, 10H)

(3) 2-[$N_\alpha$-(1-S-Carbethoxy-3-phenyl-propyl)-$N_\varepsilon$-benzyloxycarbonyl-S-lysyl]-cis,endo-2-azabicyclo[3.3.0]-octan-3-S-carbonsäurebenzylester

a) 560 mg 2-Azabicyclo[3.3.0]octan-3-carbonsäurebenzylester-hydrochlorid, hergestellt nach dem Referenzbeispiel (3), werden mit 940 mg $N_\alpha$-(1-S-Carbethoxy-3-phenyl-propyl)-$N_\varepsilon$-benzyloxycarbonyl-S-lysin, hergestellt nach Beispiel VI (2), analog Referenzbeispiel (4) umgesetzt. Man erhält nach der Aufarbeitung 1,5 g Öl, das ein Gemisch zweier diasteromerer Verbindungen ist.

Das Diasteromerengemisch wird säulenchromatographisch mit Kieselgel und Cyclohexan/Essigester 2:1 als Elutionsmittel in die Einzelkomponenten getrennt. Das zuerst eluierte Isomere stellt obige Verbindungen dar. Es werden 0,6 g Öl erhalten.

$^1$N—NMR ($CDCl_3$) (nach H/D-Austausch mit $D_2O$): 1,0—2,6 (m, 20H); 2,6—4,5 (m, 8H); 4,6—5,0 (m, 2H); 5,1—5,3 (doppeltes s., 4H); 7,1—7,6 (m, 15H)

b) Das spätere Eluat liefert 0,4 g 2-[$N_\alpha$-(1-S-Carbethoxy-3-phenyl-propyl)-$N_\varepsilon$-benzyloxycarbonyl-S-lysyl]-cis,endo-2-azabicyclo[3.3.0]octan-3-R-carbonsäure-benzylester

$^1$H—NMR ($CDCl_3$) (nach H/D-Austausch mit $D_2O$): 1,0—2,6 (m, 20H); 2,6—4,4 (m, 8H); 4,5—5,0 (m, 2H); 5,1—5,3 (doppeltes s., 4H); 7,1—7,5 (m, 15H)

(4) 2-[$N_\alpha$-(1-S-Carbethoxy-3-phenyl-propyl)-S-lysyl]-cis,endo-2-azabicyclo[3.3.0]octan-3-S-carbonsäure-dihydrochlorid

500 mg 2-[$N_\alpha$-(1-S-Carboethoxy-3-phenyl-propyl)-$N_\varepsilon$-benzyl-oxycarbonyl-S-lysyl]-cis,endo-2-aza-bicyclo[3.3.0]octan-3-S-carbonsäurebenzylester aus Beispiel VI (3a) werden in 20 ml Ethanol gelöst und unter Zusatz von 0,1 g 10%ig Pd/C bei Normaldruck hydrogenolisch entbenzyliert. Nach Beeindigung der Wasserstoffaufnahme wird vom Katalysator abfilitriert, die ethanolische Lösung mit ethanolischer Chlorwasserstofflösung bis pH 1 versetzt, das Ethanol im Vakuum abgedampft. Der Rückstand wird mit Diisopropylether versetzt, wobei das Produkt fest wird. Es werden 200 mg erhalten.

$^1$H—NMR des Betains ($CDCl_3$, nach D/D-Austausch mit $D_2O$): 1,0—2,5 (m, 20H); 2,6—4,4 (m, 8H); 4,4—5,1 (m, 2H); 7,2 (s, 5H)

## Beispiel VII

2-[$N_\alpha$-(1-S-Carbethoxy-3-phenyl-propyl)-S-lysyl]-cis,endo-2-azabicyclo[3.3.0]octan-3-R-carbonsäure-dihydrochlorid

0,3 mg des entsprechenden Benzylesters aus Beispiel VI (3b) werden analog Beispiel VI (4) umgesetzt und aufgearbeitet. Es werden 110 mg der Carbonsäure als Dihydrochlorid erhalten.

$^1$H—NMR des Betains ($CDCl_3$, nach H/D-Austausch mit $D_2O$): 1,0—2,6 (m, 20H); 2,6—4,4 (m, 8H); 4,1—5,1 (m, 2H); 7,2 (s, 5H)

## Beispiel VIII

2-[$N_\alpha$-(1-S-Carboxy-3-phenyl-propyl)-S-lysyl]-cis,endo-2-azabicyclo[3.3.0]octan-3-S-carbonsäure-dihydrochlorid

0,5 g [$N_\alpha$-(1-S-Carbethoxy-3-phenyl-propyl)-S-lysyl]-cis,endo-2-azabicyclo[3.3.0]octan-3-S-carbonsäure-dihydrochlorid aus Beispiel VI (4) werden in 20 ml DimethoxeVI (4) werden in 20 ml Dimethoxyethan suspendiert. Es wird wäßrige 4 n KOH zugegeben bis ein pH von 9—10 erreicht ist. Es wird eine halbe

Stunde gerührt. Danach stellt man mit Salzsäure auf pH 4 ein, engt im Vakuum zur Trockene ein, nimmt den Rückstand in Essigester auf und filtriert. Die Essigesterlösung wird eingeengt, der Rückstand wird mit Diisopropylether verrieben, wobei er fest wird.

Ausbeute: 0,35 g

$^1H$—NMR ($D_2O$): 1,2—2,5 (m, 17H); 2,5—4,5 (m, 6H); 4,5—5,0 (m, 2H); 7,2 (s, 5H)

Beispiel IX

2-[$N_\alpha$-(1-S-Carboxy-3-phenyl-propyl)-S-lysyl]-cis,endo-2-azabicyclo[3.3.0]octan-3-R-carbonsäure-hydrochlorid

500 mg 2-[$N_\alpha$-(1-S-Carboethoxy-3-phenyl-propyl)-2-lysyl]-cis,endo-2-azabicyclo[3.3.0]octan-3-R-carbonsäure-hydrochlorid aus Beispiel VII werden analog Beispiel VIII verseift und aufgearbeitet.

Ausbeute: 0,32 g

$^1H$—NMR ($D_2O$): 1,2—2,5 (m, 17H); 2,5—4,5 (m, 6H); 4,5—5,0 (m, 2H); 7,2 (s, 5H)

Beispiel X

2-[N-(1-S-Carbethoxy-3-phenyl-propyl)-O-ethyl-S-tyrosyl]-cis,endo-2-azabicyclo[3.3.0]octan-3-S-carbonsäure

(1) N-(1-R,S-Carbethoxy-3-phenyl-propyl)-O-ethyl-S-tyrosin-benzylester

Man setzt analog Beispiel II 24 g Benzoylacrylsäureethylester in 100 ml Ethanol mit 30 g O-Ethyl-S-tyrosin-benzylester in Anwesenheit von 0,5 ml Triethylamin um und erhält nach Einengen der Lösung und Digerieren des Rückstandes mit Diethylether/Petrolether (1:1) und Trocknen im Vakuum 42 g der RS,S-Verbindung.

(2) N-(1-R,S-Carbethoxy-3-phenyl-propyl)-O-ethyl-S-tyrosin

40 g der nach X (1) erhaltenen Verbindung werden in 800 ml Essigsäure mit 4 g Pd(C) (10%) bei 100 bar und Raumtemperatur hydriert. Ausbeute nach Chromatographie an Kieselgel im Lösungsmittel Essigester/Cyclohexan (1:3) und Trocknen des Eindampfrückstandes: 25 g dünnschicht-chromatographisch nahezu einheitliche Titelverbindung.

Schmp. 205—213°C.

| $C_{23}H_{29}NO_5$ (399,5) | | C | H | N |
|---|---|---|---|---|
| | Ber. | 69,15 | 7,31 | 3,50 |
| | Gef. | 69,5 | 7,4 | 3,3 |

(3) 2-[N-(1-S-Carbethoxy-3-phenyl-propyl)-O-ethyl-S-tyrosyl]-cis,endo-2-azabicyclo[3.3.0]octan-3-S-carbonsäure

Man setzt analog dem Referenzbeispiel (4) 5 g des nach Referenzbeispiel (3) und Ausschütteln mit Diethylether aus alkalischer Lösung erhaltenen freien Benzylesters mit 8 g der nach X (2) erhaltenen Verbindung mittels 4,4 g Dicyclohexylcarbodiimid in Anwesenheit von 2,7 g 1-Hydroxy-benzotriazol um. Nach Durchführung der unter Referenzbeispiel (4) beschriebenen Chromatographie erhält man 2,9 g öligen Benzylester als Zwischenprodukt. Die $^1H$—NMR— und die Massenspektren sind im Einklang mit der angegebenen Struktur.

Der Benzylester wird in 50 ml Ethanol unter Normaldruck an Pd(C) katalytisch hydriert. Nach Abfiltrieren des Katalysators und Abdestillieren des Lösungsmittels bleibt ein fester Rückstand zurück, der mit Diethylether/Petrolether digeriert und getrocknet wird.

Ausbeute: 2,2 g.

$^1H$—NMR ($CDCl_3$): 1,2—3,0 (m, 15H); 1,27 (t, 3H); 1,4 (t, 3H); 3,0—4,3 (m, 4H); 3,8—4,2 (m 4H); 6,5—7,1 (2d, 4H); 7,3 (s, 5H)

Beispiel XI

2-[N-(1-S-Carbethoxy-3-phenyl-propyl)-O-methyl-S-tyrosyl]-cis,endo-2-azabicyclo[3.3.0]octan-3-S-carbonsäure

Man arbeitet wie in Beispiel X beschrieben, setzt aber in der X (1) analogen Stufe O-Methyl-S-tyrosinbenzyl-ester ein und erhält die Titelverbindung, deren $^1H$—NMR-Spektrum im Einklang mit der angegebenen Struktur ist.

**Patentansprüche für die Vertragsstaaten: BE CH DE FR GB IT LI LU NL SE**

1. Verbindung der Formel I

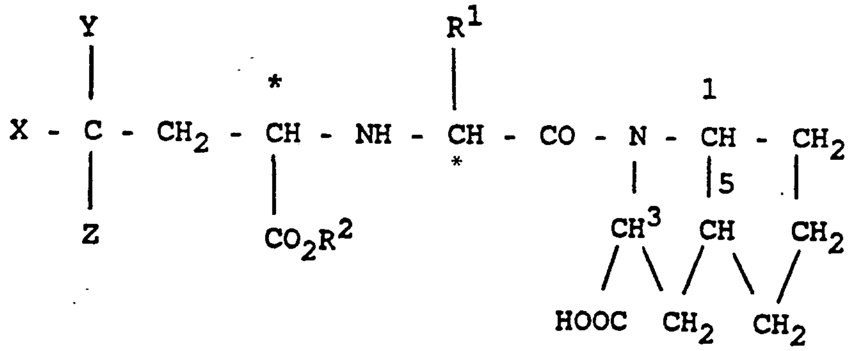

(I)

in der die Wasserstoffatome an den Brückenkopf-C-Atomen 1 und 5 zueinander cis-konfiguriert sind und die Carboxygruppe am C-Atom 3 endoständig zum bicyclischen Ringsystem orientiert (d.h. dem Cyclopentanring zugewandt) ist und in der

$R^1$ = Wasserstoff, Allyl, Vinyl oder eine Seitenkette einer gegebenenfalls geschützten natürlich vorkommenden α-Aminosäure $R^1$—CH($NH_2$)—COOH;

$R^2$ = Wasserstoff, ($C_1$—$C_6$)-Alkyl, ($C_2$—$C_6$)-Alkenyl, Aryl-($C_1$—$C_4$)-alkyl oder Nitrobenzyl,

Y = Wasserstoff oder Hydroxy,

Z = Wasserstoff oder

Y und Z = zusammen Sauerstoff,

X ($C_1$—$C_6$)-Alkyl, ($C_2$—$C_6$)-Alkenyl, ($C_5$—$C_9$)-Cycloalkyl, ($C_6$—$C_{12}$)-Aryl, das durch ($C_1$—$C_4$)-Alkyl, ($C_1$—$C_4$)Alkoxy, Hydroxy, Halogen, Nitro, Amino, ($C_1$—$C_4$)-Alkylamino, Di-($C_1$—$C_4$)-alkyl-amino oder Methylendioxy mono-, di- oder trisubstituiert sein kann, oder Indol-3-yl bedeuten.

sowie deren physiologisch unbedenkliche Salze, wobei Verbindungen der Formel I ausgenommen sind, in der die Chiralitätszentren an dem mit einem Stern (*) markierten beiden C-Atomen der Kette und am C-Atom 3 des Bicyclus in der S-Konfiguration vorliegen und in der

$R^1$ Methyl,

$R^2$ Wasserstoff, Methyl, Ethyl oder Benzyl,

Y Wasserstoff,

Z Wasserstoff und

X Phenyl bedeuten.

2. Verbindung der Formel I gemäß Anspruch 1, in welcher

$R^1$ = Methyl, die gegebenenfalls acylierte Seitenkette von Lysin oder die O-alkylierte Seitenkette von Tyrosin,

$R^2$ = Wasserstoff, Methyl, Ethyl oder Benzyl,

X = Phenyl oder mit Fluor und/oder Chlor mono- oder disubstituiertes Phenyl,

Y Wasserstoff oder Hydroxy

Z Wasserstoff oder

Y und Z zusammen Sauerstoff

bedeuten.

3. N-α-(1-S-Carbethoxy-3-phenyl-propyl)-S-lysyl-cis,endo-2-azabicyclo[3.3.0]octan-3-S-carbonsäure sowie deren physiologisch unbedenklichen Salze.

4. N-α-(1-S-Carboxy-3-phenyl-propyl)-S-lysyl-cis,endo-2-azabicyclo[3.3.0]octan-3-S-carbonsäure sowie deren physiologisch unbedenklichen Salze.

5. N-(1-S-Carbethoxy-3-phenyl-propyl)-O-ethyl-S-tyrosyl-cis,endo-2-azabicyclo[3.3.0]octan-3-S-carbonsäure sowie deren physiologisch unbedenklichen Salze.

6. N-(1-S-Carbethoxy-3-phenyl-propyl)-O-methyl-S-tyrosyl-cis,endo-2-azabicyclo[3.3.0]octan-3-S-carbonsäure sowie deren physiologisch unbedenklichen Salze.

7. Verfahren zur Herstellung einer Verbindung gemaß einem der Ansprüche 1—6, dadurch gekennzeichnet, daß man eine Verbindung der Formel II

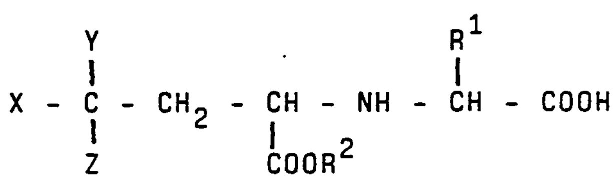

(II)

in der X, Y, Z, $R^1$ und $R^2$ die im Anspruch 1 definierten Bedeutungen mit Ausnahme von $R^2$ = Wasserstoff haben, mit einer Verbindung der Formeln IIIa oder IIIb

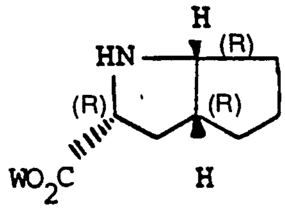 (IIIa) 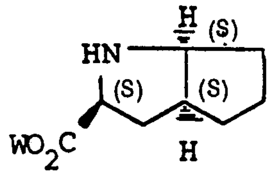 (IIIb)

in der W eine Carboxy veresternde Gruppe bedeutet, umsetzt und anschließend das Produkt hydriert oder mit einer Säure oder einer Base behandelt und die erhaltene Verbindung gegebenenfalls in ihr physiologisch verträgliches Salz überführt.

8 Pharmazeutisches Mittel enthaltend eine Verbindung gemäß einem der Ansprüche 1—6.

9. Verbindung gemäß einem der Ansprüche 1—6 zur Anwendung als Heilmittel.

10. Verbindung gemäß einem der Ansprüche 1—6 als Heilmittel zur Anwendung bei der Behandlung des Bluthochdrucks.

11. Mittel gemäß Anspruch 8, dadurch gekennzeichnet, daß es ein Diuretikum enthält.

12. Verbindung gemäß Anspruch 9 oder 10 zur Anwendung in Kombination mit einem Diuretikum.

13. Verfahren zur Herstellung einer Verbindung der Formel II, gemäß Anspruch 7, in welcher X, $R^1$ und $R^2$ die in Anspruch 1 definierten Bedeutungen haben, Y Wasserstoff oder Hydroxy und Z Wasserstoff bedeuten, dadurch gekennzeichnet, daß man

a) falls X Aryl ist, Arylmethylketone $X—CO—CH_3$ mit Glyoxylsäureestern $CHO—CO_2R^2$ und α-Aminosäureestern $H_2N—CH(R^1)—CO_2W'$, in welcher W' ein hydrogenolytisch, basisch oder sauer abspaltbarer Rest ist und $R^2$ für den Fall W' = Benzyl nicht Benzyl oder Nitrobenzyl bedeutet, zu Verbindungen der Formel IV umsetzt

$$X - CO - CH_2 - \underset{\displaystyle CO_2R^2}{\overset{|}{CH}} - NH - \underset{\displaystyle R^1}{\overset{|}{CH}} - CO_2W' \qquad (IV)$$

in der X, W', $R^1$ und $R^2$ die vorstehend angegebenen Bedeutungen haben, oder

b) Keto-Acrylsäureester $X—CO—CH{=}CH—CO_2R^2$ mit α-Aminosäureestern $H_2N—CH(R^1)—CO_2W'$ zu Verbindungen der Formel IV umsetzt,

diese nach a) oder b) erhaltenen Verbindungen, falls W' ein sauer oder basisch abspaltbarer Rest ist, gegebenenfalls einer sauren oder basischen Esterspaltung unterwirft oder, falls W' = Benzyl oder Nitrobenzyl ($R^2$ = Benzyl oder Nitrobenzyl) hydrogenolytisch in die Carbonsäure überführt und zu Verbindungen der Formel II, in der der X, $R^1$ und $R^2$ die vorstehende Bedeutung hat, Y Wasserstoff oder Hydroxy und Z Wasserstoff bedeuten, beziehungsweise deren Niederalkylestern hydriert, welche gegebenenfalls auch einer sauer katalysierten Esterspaltung unterworfen werden können.

14. Verbindung der Formel IV, in der X, $R^1$ und $R^2$ die in Anspruch 1 definierten Bedeutungen haben, W' ein hydrogenlytisch, basisch oder sauer abspaltbarer Rest bedeutet und $R^2$ für den Fall W' = Benzyl nicht Benzyl oder Nitrobenzyl ist.

**Patentansprüche für den Vertragsstaat: AT**

1. Verfahren zur Herstellung einer Verbindung der Formel I

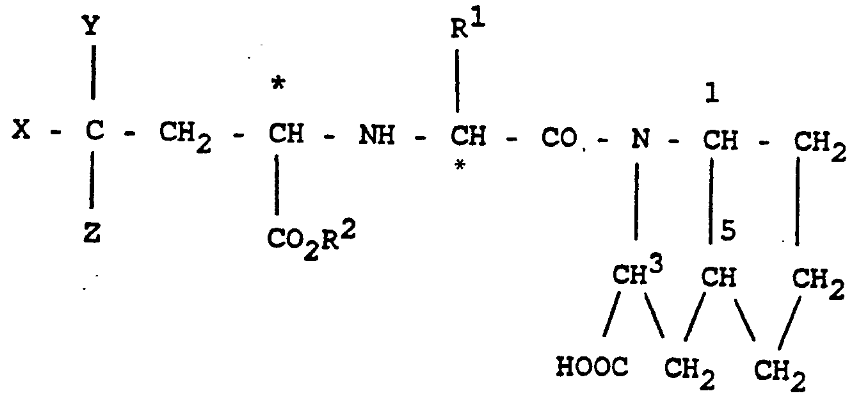

(I)

in der die Wasserstoffatome an den Brückenkopf-C-Atomen 1 und 5 zueinander cis-konfiguriert sind und die Carboxygruppe am C-Atom 3 endoständig zum bicyclischen Ringsystem orientiert (d.h. dem Cyclopentanring zugewandt) ist und der

$R^1$ = Wasserstoff, Allyl, Vinyl oder eine Seitenkette einer gegebenenfalls geschützten natürlich vorkommenden α-Aminosäure $R^1$—$CH(NH_2)$—COOH,

$R^2$ = Wasserstoff, ($C_1$—$C_6$)-Alkyl, ($C_2$—$C_6$)-Alkenyl, Aryl-($C_1$—$C_4$)-alkyl oder Nitrobenzyl,

Y = Wasserstoff oder Hydroxy,

Z = Wasserstoff oder

Y und Z = zusammen Sauerstoff,

X = ($C_1$—$C_6$)-Alkyl, ($C_2$—$C_6$)-Alkenyl, ($C_5$—$C_9$)-Cycloalkyl, ($C_6$—$C_{12}$)-Aryl, das durch ($C_1$—$C_4$)-Alkyl, ($C_1$—$C_4$)-Alkoxy, Hydroxy, Halogen, Nitro, Amino, ($C_1$—$C_4$)-Alkylamino, Di-($C_1$—$C_4$)-alkyl-amino oder Methylendioxy mono-, di- oder trisubstituiert sein kann, oder Indol-3-yl bedeuten,

sowie deren physiologisch unbedenklichen Salze, wobei Verbindungen der Formel I ausgenommen sind, in der die Chiralitätszentren an dem mit einem Stern (*) markierten beiden C-Atomen der Kette und am C-Atom 3 des Bicyclus in der S-Konfiguration vorliegen und in der

$R^1$ = Methyl,

$R^2$ = Wasserstoff, Methyl, Ethyl oder Benzyl,

Y = Wasserstoff,

Z = Wasserstoff und

X = Phenyl bedeuten

dadurch gekennzeichnet, daß man eine Verbindung der Formel II

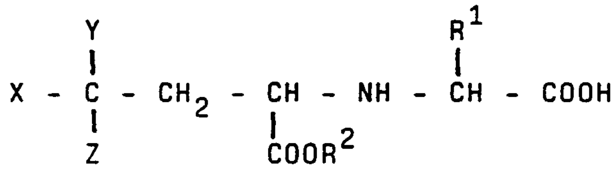

(II)

in der X, Y, Z, $R^1$ und $R^2$ die oben definierten Bedeutungen mit Ausnahme von $R^2$ = Wasserstoff haben, mit einer Verbindung der Formeln IIIa und IIIb

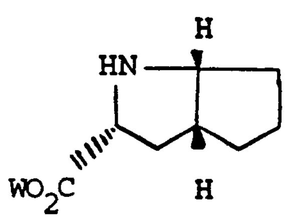

(IIIa)

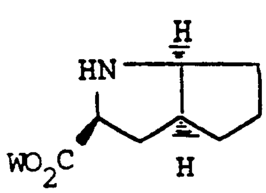

(IIIb)

in deren W eine Carboxy veresternde Gruppe bedeutet, umsetzt und anschließend das Produkt hydriert oder mit einer Säure oder einer Base behandelt und die erhaltene Verbindung gegebenenfalls in der physiologisch verträgliches Salz überführt.

2. Verfahren gemäß Anspruch 1, dadurch gekennzeichnet, daß in Formel I

$R^1$ = Methyl, die gegebenenfalls acylierte Seitenkette von Lysin oder die O-alkylierte Seitenkette von Tyrosin,
$R^2$ = Wasserstoff, Methyl, Ethyl oder Benzyl,
X = Phenyl oder mit Fluor und/oder Chlor mono- oder disubstituiertes Phenyl,
Y Wasserstoff oder Hydroxy
Z Wasserstoff oder
Y und Z zusammen Sauerstoff
bedeuten.

3. Verfahren gemäß Anspruch 1, dadurch gekennzeichnet, daß N-α-(1-S-Carbethoxy-3-phenyl-propyl)-S-lysyl-cis,endo-2-azabicyclo[3.3.0]octan-3-S-carbonsäure oder ihr physiologisch verträgliches Salz hergestellt wird.

4. Verfahren gemäß Anspruch 1, dadurch gekennzeichnet, daß N-α-(1-S-Carboxy-3-phenyl-propyl)-S-lysyl-cis,endo-2-azabicyclo[3.3.0]octan-3-S-carbonsäure oder ihr physiologisch verträgliches Salz hergestellt wird.

5. Verfahren gemäß Anspruch 1, dadurch gekennzeichnet, daß N-(1-S-Carbethoxy-3-phenyl-propyl)-O-ethyl-S-tyrosyl-cis,endo-2-azabicyclo[3.3.0]octan-3-S-carbonsäure oder ihr physiologisch verträgliches Salz hergestellt wird.

6. Verfahren gemäß Anspruch 1, dadurch gekennzeichnet, daß N-(1-S-Carbethoxy-3-phenyl-propyl)-O-methyl-S-tyrosyl-cis,endo-2-azabicyclo[3.3.0]octan-3-S-carbonsäure oder ihr physiologisch verträgliches Salz hergestellt wird.

7. Verfahren zur Herstellung eines pharmazeutischen Mittels enthaltend eine Verbindung der Formel I gemäß einem der Ansprüche 1—6, dadurch gekennzeichnet, daß man diese in eine geeignete Darreichungsform bringt.

8. Verfahren gemäß Anspruch 1 zur Herstellung einer Verbindung der Formel I zur Anwendung als Heilmittel.

9. Verfahren gemäß Anspruch 1 zur Herstellung einer Verbindung der Formel I als Heilmittel zur Anwendung bei der Behandlung des Bluthochdruckes.

10. Verfahren gemäß Anspruch 7, dadurch gekennzeichnet, daß man ein Diuretikum zusetzt.

11. Verfahren zur Herstellung von Verbindungen der Formel II, gemäß Anspruch 1, in welcher X, $R^1$ und $R^2$ die in Anspruch 1 definierten Bedeutungen haben, Y Wasserstoff oder Hydroxy und Z Wasserstoff bedeuten, dadurch gekennzeichnet, daß man

a) falls X Aryl ist, Arylmethylketone X—CO—$CH_3$ mit Glyoxylsäureestern CHO—$CO_2R^2$ und α-Aminosäureestern $H_2N$—$CH(R^1)$—$CO_2W'$, in welcher W' ein hydrogenolytisch, basisch oder sauer abspaltbarer Rest ist und $R^2$ für den Fall W' = Benzyl nicht Benzyl oder Nitrobenzyl bedeutet, zu Verbindungen der Formel IV umsetzt

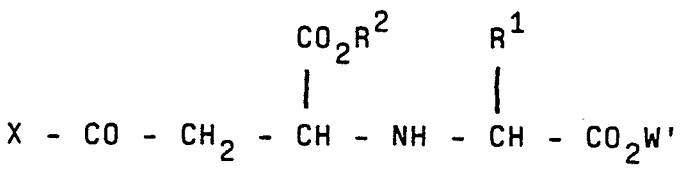
(IV)

in der X, W', $R^1$ und $R^2$ die vorstehend angegebenen Bedeutungen haben oder

b) Keto-Acrylsäureester X—CO—CH=CH—$CO_2R^2$ mit α-Aminosäureestern $H_2N$—$CH(R^1)$—$CO_2W'$ zu Verbindungen der Formel IV umsetzt,

diese nach a) oder b) erhaltenen Verbindungen, falls W' ein sauer oder basisch abspaltbarer Rest ist, gegebenenfalls einer sauren oder basischen Esterspaltung unterwirft oder, falls W' = Benzyl oder Nitrobenzyl ($R^2$ ≠ Benzyl oder Nitrobenzyl) hydrogenolytisch in die Carbonsäure überführt und zu Verbindungen der Formel II, in der der X, $R^1$ und $R^2$ die vorstehende Bedeutung hat, Y Wasserstoff oder Hydroxy und Z Wasserstoff bedeuten, beziehungsweise deren Niederalkylestern hydriert, welche gegebenenfalls auch einer sauer katalysierten Esterspaltung unterworfen werden können.

**Claims for the Contracting States: BE CH DE FR GB IT LI LU NL SE**

1. A compound of the formula I

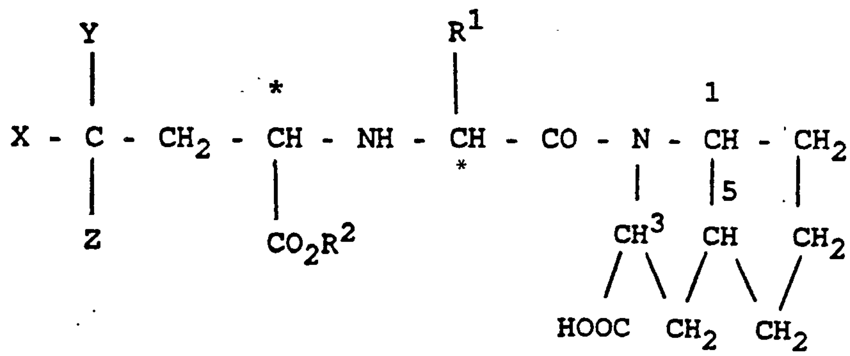

(I)

in which the hydrogen atoms on the bridge-head carbon atoms 1 and 5 are in the cis-configuration relative to one another and the carboxyl group on carbon atom 3 is orientated in the endo-position relative to the bicyclic ring system (i.e. towards the cyclopentane ring), and in which

$R^1$ denotes hydrogen, allyl, vinyl or a side-chain of a naturally occurring α-aminoacid $R^1$—$CH(NH_2)$—COOH, which may be protected,

$R^2$ denotes hydrogen, $(C_1—C_6)$-alkyl, $(C_2—C_6)$-alkenyl, aryl-$(C_1—C_4)$-alkyl or nitrobenzyl,

Y denotes hydrogen or hydroxyl and

Z denotes hydrogen, or

Y and Z together denote oxygen, and

X denotes $(C_1—C_5)$-alkyl, $(C_2—C_6)$-alkenyl, $(C_5—C_9)$-cycloalkyl, $(C_6—C_{12})$-aryl, which can be mono-, di- or tri-substituted by $(C_1—C_4)$-alkyl, $(C_1—C_4)$-alkoxy, hydroxyl, halogen, nitro, amino, $(C_1—C_4)$-alkylamino, di-$(C_1—C_4)$-alkylamino or methylenedioxy, or denotes indol-3-yl,

and physiologically acceptable salts thereof, compounds of the formula I being excluded in which the centres of chirality on the two carbon atoms labelled with a star (*) in the chain and on carbon atom 3 of the bicyclic ring system are present in the S-configuration and in which

$R^1$ denotes methyl,

$R^2$ denotes hydrogen, methyl, ethyl or benzyl,

Y denotes hydrogen,

Z denotes hydrogen and

X denotes phenyl.

2. A compound of the formula I as claimed in claim 1, in which

$R^1$ denotes methyl, the side chain of lysine, which may be acylated, or the O-alkylated side-chain of tyrosine,

$R^2$ denotes hydrogen, methyl, ethyl or benzyl,

X denotes phenyl, or phenyl which is mono- or di-substituted by fluorine and/or chlorine,

Y denotes hydrogen or hydroxyl and

Z denotes hydrogen, or

Y and Z together denote oxygen.

3. N-α-(1-S-Carbethoxy-3-phenyl-propyl)-S-lysyl-cis,endo-2-azabicyclo[3.3.0]octane-3-S-carboxylic acid and physiologically acceptable salts thereof.

4. N-α-(1-S-Carboxy-3-phenyl-propyl)-S-lysyl-cis,endo-2-azabicyclo[3.3.0]octane-3-S-carboxylic acid and physiologically acceptable salts thereof.

5. N-(1-S-Carbethoxy-3-phenyl-propyl)-O-ethyl-S-tyrosyl-cis,endo-2-azabicyclo[3.3.0]octane-3-S-carboxylic acid and physiologically acceptable salts thereof.

6. N-(1-S-Carbethoxy-3-phenyl-propyl)-O-methyl-S-tyrosyl-cis,endo-2-azabicyclo[3.3.0]octane-3-S-carboxylic acid and physiologically acceptable salts thereof.

7. A process for the preparation of a compound as claimed in one of claims 1—6, which comprises reacting a compound of the formula II

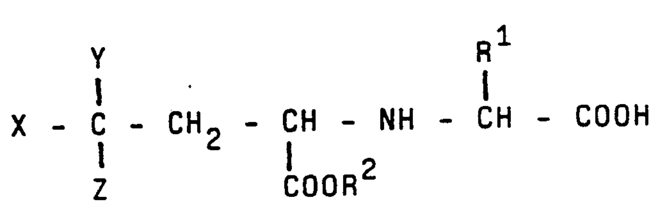

(II)

in which X, Y, Z, $R^1$ and $R^2$ have the meanings defined in claim 1, with the exception that $R^2$ cannot be hydrogen, with a compound of the formula IIIa or IIIb

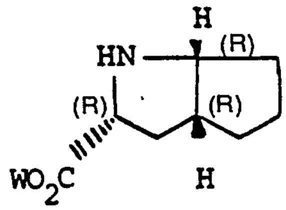 (IIIa) 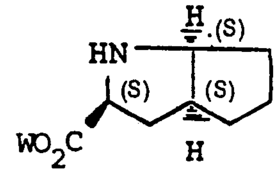 (IIIb)

in which W denotes a carboxyl-esterifying group, and then hydrogenating the product or treating it with an acid or a base and, if appropriate, converting the compound obtained into its physiologically tolerated salt.

8. A pharmaceutical agent containing a compound as claimed one of in claims 1—6.

9. A compound as claimed in one of claims 1—6 for use as a medicine.

10. A compound as claimed in one of claims 1—6 as a medicine for use in the treatment of high blood pressure.

11. An agent as claimed in claim 8, which contains a diuretic agent.

12. A compound as claimed in claim 9 or 10 for use in combination with a diuretic agent.

13. The process for the preparation of a compound of the formula II as claimed in claim 7, in which X, $R^1$ and $R^2$ have the meanings defined in claim 1, Y denotes hydrogen or hydroxyl and Z denotes hydrogen, which comprises

a) if X is aryl, reacting an aryl methyl ketone X—CO—$CH_3$ with a glyoxylic acid ester CHO—$CO_2R^2$ and an α-aminoacid ester $H_2N$—CH($R^1$)—$CO_2W'$, in which W' is a radical which can be split off by hydrogenolysis or treatment with a base or an acid and $R^2$ does not denote benzyl or nitrobenzyl if W' is benzyl, to give a compound of the formula IV

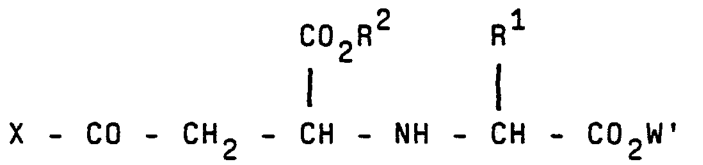 (IV)

in which X, W', $R^1$ and $R^2$ have the meanings given above, or

b) reacting a keto-acrylic acid ester X—CO—CH=CH—$CO_2R^2$ with an α-aminoacid ester $H_2N$—CH($R^1$)—$CO_2W'$ to give a compound of the formula IV,

if appropriate subjecting this compound obtained according to a) or b) if W' is a radical which can be split off under acidic or basic conditions, to acidic or basic ester cleavage, or, if W' is benzyl or nitrobenzyl ($R^2 \neq$ benzyl or nitrobenzyl), converting this compound into the carboxylic acid by hydrogenolysis and hydrogenating the product to a compound of the formula II in which X, $R^1$ and $R^2$ have the above meanings, Y denotes hydrogen or hydroxyl and Z denotes hydrogen, or a lower alkyl ester thereof, which, if appropriate, can also be subjected to acid-catalyzed ester cleavage.

14. A compound of the formula IV in which X, $R^1$ and $R^2$ have the meanings defined in claim 1, W' denotes a radical which can be split off by basic or acidic hydrogenolysis and $R^2$ is not benzyl or nitrobenzyl if W' is benzyl.

**Claims for the Contracting State: AT**

1. A process for the preparation of a compound of the formula I

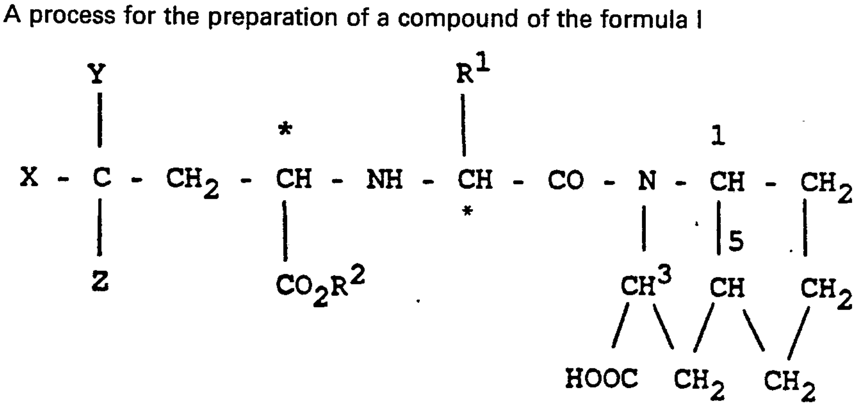 (I)

in which the hydrogen atoms on the bridge-head carbon atoms 1 and 5 are in the cis-configuration relative to one another and the carboxyl group on carbon atom 3 is orientated in the endo-position relative to the bicyclic ring system (i.e. towards the cyclopentane ring), and in which

$R^1$ denotes hydrogen, allyl, vinyl or a side-chain of a naturally occurring α-aminoacid $R^1$—CH($NH_2$)—COOH, which may be protected,

$R^2$ denotes hydrogen, ($C_1$—$C_6$)-alkyl, ($C_2$—$C_6$)-alkenyl, aryl-($C_1$—$C_4$)-alkyl or nitrobenzyl,

Y denotes hydrogen or hydroxyl and

Z denotes hydrogen, or

Y and Z together denote oxygen, and

X denotes ($C_1$—$C_5$)-alkyl, ($C_2$—$C_6$)-alkenyl, ($C_5$—$C_9$)-cycloalkyl, ($C_6$—$C_{12}$)-aryl, which can be mono-, di- or tri-substituted by ($C_1$—$C_4$)-alkyl, ($C_1$—$C_4$)-alkoxy, hydroxyl, halogen, nitro, amino, ($C_1$—$C_4$)-alkylamino, di-($C_1$—$C_4$)-alkylamino or methylenedioxy, or denotes indol-3-yl,

and physiologically acceptable salts thereof, compounds of the formula I being excluded in which the centres of chirality on the two carbon atoms labelled with a star (*) in the chain and on carbon atom 3 of the bicyclic ring system are present in the S-configuration and in which

$R^1$ denotes methyl,

$R^2$ denotes hydrogen, methyl, ethyl or benzyl,

Y denotes hydrogen,

Z denotes hydrogen and

X denotes phenyl

which comprises reacting a compound of the formula II

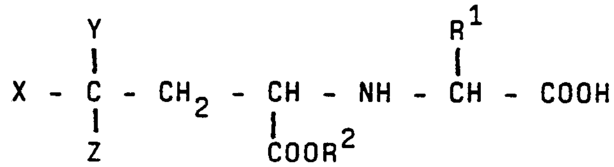

(II)

in which X, Y, Z, $R^1$ and $R^2$ have the meanings defined above, with the exception that $R^2$ cannot be hydrogen, with a compound of the formula IIIa and IIIb

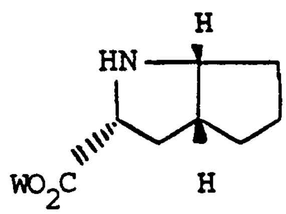

(IIIa)

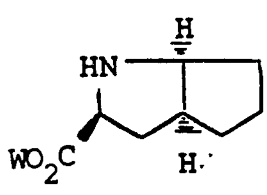

(IIIb)

in which W denotes a carboxyl-esterifying group, and then hydrogenating the product or treating it with an acid or a base and, if appropriate, converting the compound obtained into the physiologically tolerated salt.

2. The process as claimed in claim 1, wherein in the formula I

$R^1$ denotes methyl, the side-chain of lysine, which may be acylated, or the O-alkylated side chain of tyrosine,

$R^2$ denotes hydrogen, methyl, ethyl or benzyl,

X denotes phenyl, or phenyl which is mono- or di-substituted by fluorine and/or chlorine,

Y denotes hydrogen or hydroxyl and

Z denotes hydrogen, or

Y and Z together denote oxygen.

3. The process as claimed in claim 1, wherein N-α-(1-S-carbethoxy-3-phenyl-propyl)-S-lysyl-cis,endo-2-azabicyclo[3.3.0]octane-3-S-carboxylic acid or its physiologically tolerated salt is prepared.

4. The process as claimed in claim 1, wherein N-α-(1-S-carboxy-3-phenyl-propyl)-S-lysyl-cis,endo-2-azabicyclo[3.3.0]octane-3-S-carboxylic acid or its physiologically tolerated salt is prepared.

5. The process as claimed in claim 1, wherein N-(1-S-carbethoxy-3-phenyl-propyl)-O-ethyl-S-tyrosyl-cis,endo-2-azabicyclo[3.3.0]octane-3-S-carboxylic acid or its physiologically tolerated salt is prepared.

6. The process as claimed in claim 1, wherein N-(1-S-carbethoxy-3-phenyl-propyl)-O-methyl-S-tyrosyl-cis,endo-2-azabicyclo[3.3.0]octane-3-S-carboxylic acid or its physiologically tolerated salt is prepared.

7. A process for the preparation of a pharmaceutical agent containing a compound of the formula I as claimed in one of claims 1—6, wherein said compound is brought into a suitable administration form.

8. The process as claimed in claim 1 for the preparation of a compound of the formula I for use as a medicine.

9. The process as claimed in claim 1 for the preparation of a compound of the formula I as a medicine for use in the treatment of high blood pressure.

10. The process as claimed in claim 7, wherein a diuretic agent is added.

11. A process for the preparation of a compound of the formula II, as claimed in claim 1, in which X, $R^1$

and $R^2$ have the meanings defined in claim 1, Y denotes hydrogen or hydroxyl and Z denotes hydrogen, which comprises

a) if X is aryl, reacting an aryl methyl ketone X—CO—$CH_3$ with a glyoxylic acid ester CHO—$CO_2R^2$ and an α-aminoacid ester $H_2N$—$CH(R^1)$—$CO_2W'$, in which W' is a radical which can be split off by hydrogenolysis or treatment with a base or an acid and $R^2$ does not denote benzyl or nitrobenzyl if W' is benzyl, to give a compound of the formula IV

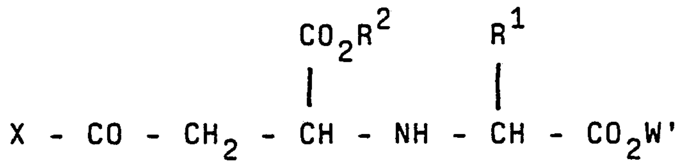

(IV)

in which X, W', $R^1$ and $R^2$ have the meanings given above, or

b) reacting a keto-acrylic acid ester X—CO—CH=CH—$CO_2R^2$ with an α-aminoacid ester $H_2N$—$CH(R^1)$—$CO_2W'$ to give a compound of the formula IV,

if appropriate subjecting this compound obtained according to a) or b) if W' is a radical which can be split off under acidic or basic conditions, to acidic or basic ester cleavage, or, if W' is benzyl or nitrobenzyl ($R^2 \neq$ benzyl or nitrobenzyl), converting this compound into the carboxylic acid by hydrogenolysis and hydrogenating the product to a compound of the formula II in which X, $R^1$ and $R^2$ have the above meanings, Y denotes hydrogen or hydroxyl and Z denotes hydrogen, or a lower alkyl ester thereof, which, if appropriate, can also be subjected to acid-catalyzed ester cleavage.

**Revendications pour les Etats contractants: BE CH DE FR GB IT LI LU NL SE**

1. Composés de formule I

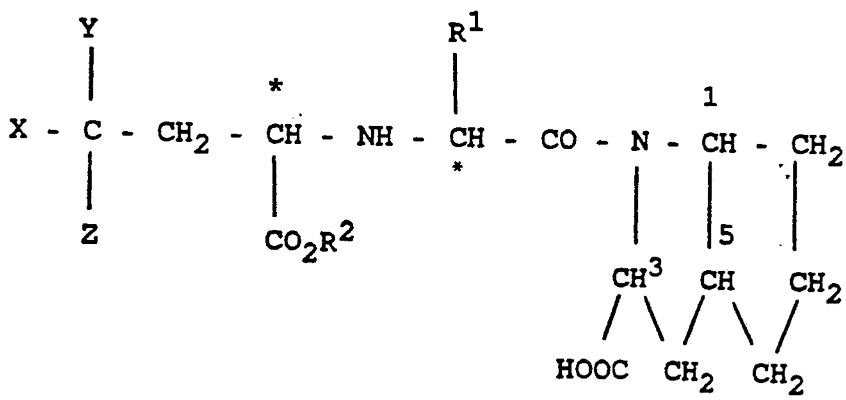

(I)

dans laquelle les atomes d'hydrogène fixés sur les atomes de carbone têtes de pont 1 et 5 ont, l'un par rapport à l'autre, la configuration cis, et le groupe carboxyle sur l'atome de carbone 3 est orienté en endo par rapport au système bicyclique (c'est-à-dire qu'il est tourné vers le cycle cyclopentane), et dans laquelle

$R^1$ est hydrogène, allyle, vinyle ou la chaîne latérale d'un acide α-aminé naturel $R^1$—$CH(NH_2)$—COOH, éventuellement protégé;

$R^2$ est hydrogène, alkyle en $C_1$—$C_6$, alkényle en $C_2$—$C_6$, arylalkyle à alkyle en $C_1$—$C_4$ ou nitrobenzyle;

Y est hydrogène ou hydroxy;

Z est hydrogène, ou

Y et Z ensemble représentent un atome d'oxygène;

X est alkyle en $C_1$—$C_6$, alkényle en $C_2$—$C_6$, cycloalkyle en $C_5$—$C_9$, aryle en $C_6$—$C_{12}$, pouvant être mono-, di- ou trisubstitué par alkyle en $C_1$—$C_4$, alcoxy en $C_1$—$C_4$, hydroxy, halogène, nitro, amino, alkylamino en $C_1$—$C_4$, dialkylamino en $C_1$—$C_4$ ou méthylènedioxy, ou indol-3-yle,

ainsi que leurs sels physiologiquement acceptables, à l'exclusion des composés de formule I et de leurs sels dans lesquels les centres de chiralité sur les deux atomes de carbone de la chaîne marqués d'une étoile (*) et sur l'atome de carbone 3 du bicycle ont la configuration S, et dans lesquels

$R^1$ est méthyle,

$R^2$ est hydrogène, méthyle, éthyle ou benzyle,

Y est hydrogène,

Z est hydrogène et
X est phényle.

2. Composé de formule I, selon la revendication 1, dans laquelle
$R^1$ est le groupe méthyle, la chaîne latérale éventuellement acylée de la lysine ou la chaîne latérale O-alkylée de la tyrosine,
$R^2$ est hydrogène, méthyle, éthyle ou benzyle,
X est phényle ou phényle mono- ou disubstitué par du fluor et/ou du chlore,
Y est hydrogène ou hydroxy,
Z est hydrogène, ou
Y et Z ensemble représentent l'oxygène.

3. Acide Nα-(1-S-carbéthoxy-3-phénylpropyl)-S-lysyl]-cis, endo-2-azabicyclo[3.3.0]octane-3-S-carboxylique ainsi que ses sels physiologiquement acceptables.

4. Acide Nα-(1-S-carboxy-3-phénylpropyl)-S-lysyl]-cis, endo-2-azabicyclo[3.3.0]octane-3-S-carboxylique ainsi que ses sels physiologiquement acceptables.

5. Acide N-(1-S-carbéthoxy-3-phénylpropyl)-O-éthyl-S-tyrosyl]-cis, endo-2-azabicyclo[3.3.0]octane-3-S-carboxylique ainsi que ses sels physiologiquement acceptables.

6. Acide N-(1-S-carbéthoxy-3-phénylpropyl)-O-méthyl-S-tyrosyl]-cis, endo-2-azabicyclo[3.3.0]octane-3-S-carboxylique ainsi que ses sels physiologiquement acceptables.

7. Procédé de préparation d'un composé selon l'une des revendications 1 à 6, caractérisé en ce que l'on fait réagir un composé de formule II

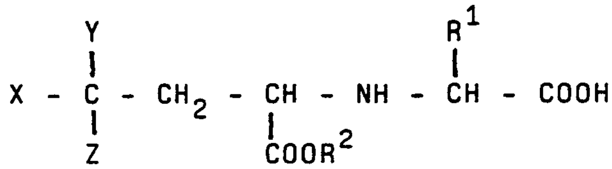

(II)

dans laquelle, X, Y, Z et $R_1$ et $R_2$ sont définis comme dans la revendication 1, à l'exception de $R_2$ = hydrogène, avec un composé de formules IIIa ou IIIb

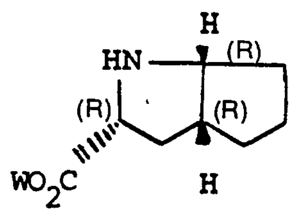

(IIIa)

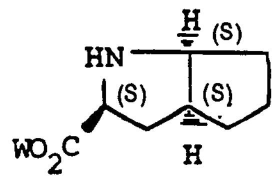

(IIIb)

dans lesquelles W est un groupe estérifiant pour le carboxy, puis qu'on hydrogène le produit ou qu'on le traite par un acide ou une base, et que l'on transforme éventuellement le composé obtenu en son sel physiologiquement acceptable.

8. Produit pharmaceutique renfermant un composé selon l'une des revendications 1 à 6.

9. Composé selon l'une des revendications 1 à 6, utilisé comme médicament.

10. Composé selon l'une des revendications 1 à 6, utilisé comme médicament pour le traitement de l'hypertension.

11. Agent selon la revendication 8, caractérisé en ce qu'il contient un diurétique.

12. Composé selon la revendication 9 ou 10, utilisé en association un diurétique.

13. Procédé de préparation, selon la revendication 7, d'un composé de formule II, dans laquelle X. $R^1$ et $R^2$ ont les définitions données dans la revendication 1, Y est hydrogène ou hydroxy et Z est hydrogène, caractérisé en ce que

a) dans la cas où X est aryle, on fait réagir des arylméthylcétones X—CO—$CH_3$ avec des esters d'acides glyoxyliques CHO—$CO_2R^2$ et des esters d'acides α-aminés $H_2N$—CH($R^1$)—$CO_2W$ dans lesquels W' est un reste détachable par hydrogénolyse on traitement par une base ou un acide, et $R^2$ n'est ni benzyle ni nitrobenzyle lorsque W' est benzyle, pour donner des composés de formule IV

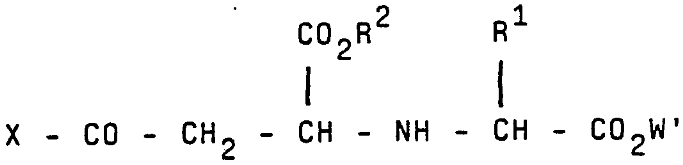

(IV)

dans laquelle X, W', $R^1$ er $R^2$ ont les significations indiquées ci-dessus, ou bien

b) on fait réagir des esters d'acides cétoacryliques X—CO—CH=CH—$CO_2R^2$ avec des esters d'acides α-aminés $H_2N$—$CH(R^1)$—$CO_2W'$ pour donner des composés de formule VI,

en ce que l'on soumet éventuellement à une saponification acide ou basique les composés obtenus selon a) ou b), dans le cas où W' est un reste détachable en milieu acide ou basique, ou bien, lorsque W' = benzyle ou nitrobenzyl ($R^2 \neq$ benzyle ou nitrobenzyle), on les transforme par hydrogénolyse en les acides carboxyliques correspondants, et on les hydrogène pour obtenir les composés de formule II, dans laquelle X, $R^1$ et $R^2$ ont les significations ci-dessus, Y est hydrogène ou hydroxy et Z est hydrogène, our leurs esters d'alkyle inférieur, qui peuvent aussi être éventuellement soumis à une saponification catalysée par un acide.

14. Composé de formule IV, dans laquelle X, $R^1$ et $R^2$ ont les significations données dans la revendication 1, W' est un reste détachable par hydrogènolyse en milieu basique ou acide et $R^2$ n'est ni benzyle ni nitrobenzyle lorsque W' = benzyle.

**Revendications pour l'Etat contractant: AT**

1. Procédé de préparation d'un composé de formule I

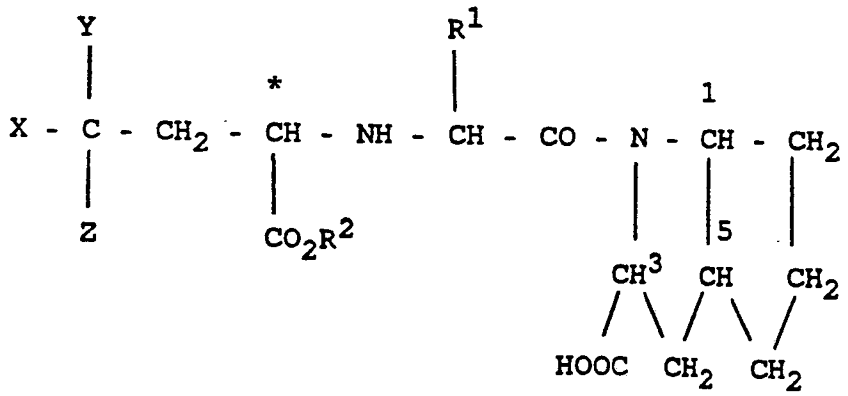

(I)

dans laquelle les atomes d'hydrogène fixés sur les atomes de carbone têtes de pont 1 et 5 ont, l'un par rapport à l'autre, la configuration cis, et le groupe carboxyle sur l'atome de carbone 3 est orienté en endo par rapport au système bicyclique (c'est-à-dire qu'il est tourné vers le cycle cyclopentane), et dans laquelle

$R^1$ est hydrogène, allyle, vinyle ou la chaîne latérale d'un acide α-aminé naturel $R^1$—$CH(NH_2)$—COOH, éventuellement protégé;

$R^2$ est hydrogène, alkyle en $C_1$—$C_6$, alkényle en $C_2$—$C_6$, arylalkyle à alkyle en $C_1$—$C_4$ ou nitrobenzyle;

Y est hydrogène ou hydroxy;

Z est hydrogène, ou

Y et Z ensemble représentent un atome d'oxygène;

X est alkyle en $C_1$—$C_6$, alkényle en $C_2$—$C_6$, cycloalkyle en $C_5$—$C_9$, aryle en $C_6$—$C_{12}$, pouvant être mono-, di- ou trisubstitué par alkyle en $C_1$—$C_4$, alcoxy en $C_1$—$C_4$, hydroxy, halogène, nitro, amino, alkylamino en $C_1$—$C_4$, dialkylamino en $C_1$—$C_4$ ou méthylènedioxy, ou indol-3-yle,

ainsi que leurs sels physiologiquement acceptables, à l'exclusion des composés de formule I et de leurs sels dans lesquels les centres de chiralité sur les deux atomes de carbone de la chaîne marqués d'une étoile (*) et sur l'atome de carbone 3 du bicycle ont la configuration S, et dans lesquels

$R^1$ est méthyle,

$R^2$ est hydrogène, méthyle, éthyle ou benzyle,

Y est hydrogène,

Z est hydrogène et

X est phényle

caractérisé en ce l'on fait réagir un composé de formule II

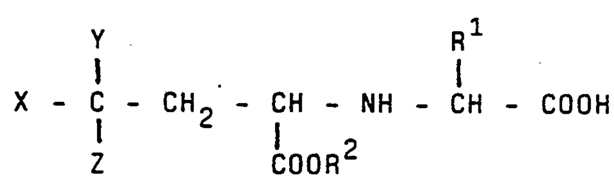

(II)

dans laquelle X, Y, Z et $R_1$ et $R_2$ sont définis comme ci-dessus, à l'exception de $R_2$ = hydrogéne, avec un composé de formules IIIa ou IIIb

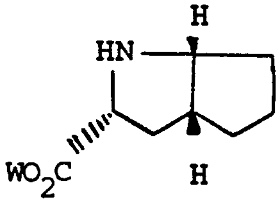 (IIIa) 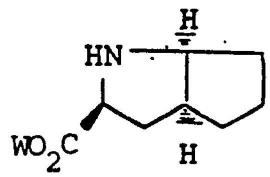 (IIIb)

dans lesquelles W est un groupe estérifiant pour le carboxy, puis qu'on hydrogène le produit ou qu'on le traite par un acide ou une base, et que l'on transforme éventuellement le composé obtenu en son sel physiologiquement acceptable.

2. Procéde selon la revendication 1, caractérise en ce que, dans la formule I,

$R^1$ est le groupe méthyle, la chaîne latérale éventuellement acylée de la lysine ou la chaîne latérale O-alkylée de la tyrosine,

$R^2$ est hydrogène, méthyle, éthyle ou benzyle,

X est phényle ou phényle mono- ou disubstitué par du fluor et/ou du chlore,

Y est hydrogène ou hydroxy,

Z est hydrogène, ou

Y et Z ensemble représentent l'oxygène.

3. Procédé selon la revendication 1, caractérisé en ce que l'on prépare l'acide Nα-(1-S-carbéthoxy-3-phénylpropyl)-S-lysyl]-cis, endo-2-azabicyclo[3.3.0]octane-3-S-carboxylique ou son sel physiologiquement acceptable.

4. Procédé selon la revendication 1, caractérisé en ce que l'on prépare l'acide Nα-(1-S-carboxy-3-phénylpropyl)-S-lysyl]-cis, endo-2-azabicyclo[3.3.0]octane-3-S-carboxylique ou son sel physiologiquement acceptable.

5. Procédé selon la revendication 1, caractérisé en ce que l'on prépare l'acide N-(1-S-carbéthoxy-3-phénylpropyl)-O-éthyl-S-tyrosyl]-cis, endo-2-azabicyclo[3.3.0]octane-3-S-carboxylique ou son sel physiologiquement acceptable.

6. Procédé selon la revendication 1, caractérisé en ce que l'on prépare l'acide N-(1-S-carbéthoxy-3-phénylpropyl)-O-méthyl-S-tyrosyl]-cis, endo-2-azabicyclo[3.3.0]octane-3-S-carboxylique ou son sel physiologiquement acceptable.

7. Procédé pour la préparation d'un produit pharmaceutique contenant un composé de formule I selon l'une des revendications 1 à 6, caractérisé en ce que l'on présente celui-ci sous une forme d'administration appropriée.

8. Procédé selon la revendication 1 pour la fabrication d'un composé de formule I utilisé comme médicament.

9. Procédé selon la revendication 1 pour la fabrication d'un composé de formule I utilisé comme médicament pour le traitement de l'hypertension.

10. Procédé selon la revendication 7, caractérisé en ce que l'on ajoute un diurétique.

11. Procédé de préparation, selon la revendication 1, de composés de formule II, dans laquelle X, $R^1$ et $R^2$ ont les définitions données dans la revendication 1, Y est hydrogène ou hydroxy et Z est hydrogène, caractérisé en ce que

a) dans le cas où X est aryle, on fait réagir des arylméthylcétones X—CO—$CH_3$ avec des esters d'acides glyoxyliques CHO—$CO_2R^2$ et des ester d'acides α-aminés $H_2N$—CH($R^1$)—$CO_2W$ dans lesquels W' est un reste détachable par hydrogénolyse on traitement par une base ou un acide, et $R^2$ n'est ni benzyle ni nitrobenzyle lorsque W' est benzyle, pour donner des composés de formule IV

$$X - CO - CH_2 - \underset{\displaystyle CO_2R^2}{\overset{|}{CH}} - NH - \underset{\displaystyle R^1}{\overset{|}{CH}} - CO_2W' \qquad (IV)$$

dans laquelle X, W', $R^1$ er $R^2$ ont les significations indiquées ci-dessus, ou bien

b) on fait réagir des esters d'acides cétoacryliques X—CO—CH=CH—$CO_2R^2$ avec des ester d'acides α-aminés $H_2N$—CH($R^1$)—$CO_2W'$ pour donner des composés de formule VI,

en ce que l'on soumet éventuellement à une saponification acide ou basique les composés obtenus selon a) ou b), dans le cas où W' est un reste détachable en milieu acide ou basique, ou bien, lorsque W' = benzyle ou nitrobenzyl ($R^2 \neq$ benzyle ou nitrobenzyle), on les transforme par hydrogénolyse en les acides carboxyliques correspondants, et on les hydrogène pour obtenir les composés de formule II, dans laquelle X, $R^1$ et $R^2$ ont les significations ci-dessus, Y est hydrogène ou hydroxy et Z est hydrogène, our leurs esters d'alkyle inférieur, qui peuvent aussi être éventuellement soumis à une saponification catalysée par un acide.